(19) Europäisches Patentamt

European Patent Office

Office européen des brevets

(11) EP 3 650 010 B1

(12) **EUROPEAN PATENT SPECIFICATION**

(45) Date of publication and mention
of the grant of the patent:
**06.01.2021 Bulletin 2021/01**

(21) Application number: **18205420.5**

(22) Date of filing: **09.11.2018**

(51) Int Cl.:
*A61K 9/14* *(2006.01)*    *A61K 9/20* *(2006.01)*
*A61K 31/00* *(2006.01)*

(54) **IMPROVED TABLETING PROCESS**

VERBESSERTER TABLETTIERUNGSPROZESS

PROCESSUS DE COMPRESSION AMÉLIORÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.05.2020 Bulletin 2020/20**

(73) Proprietor: **Siegfried AG**
**4800 Zofingen (CH)**

(72) Inventors:
• **Röhrich, Tillmann**
**4310 Rheinfelden (CH)**

• **Galea, Walter**
**Siggiewi, SGW 2121 (MT)**

(74) Representative: **Harms, Guido**
**Siegfried Hameln Services GmbH**
**Langes Feld 13**
**31789 Hameln (DE)**

(56) References cited:
**WO-A1-2013/100701    WO-A2-2006/052254
WO-A2-2007/014124    US-A1- 2011 064 820**

## Description

### Field of the invention

The present invention relates to the process of tablet manufacturing. In particular, the invention relates to the quantity of water that is needed in the final tableting process. The invention describes further how to manage different needs of water quantities during granulation, drying and tableting.

### Technical Background:

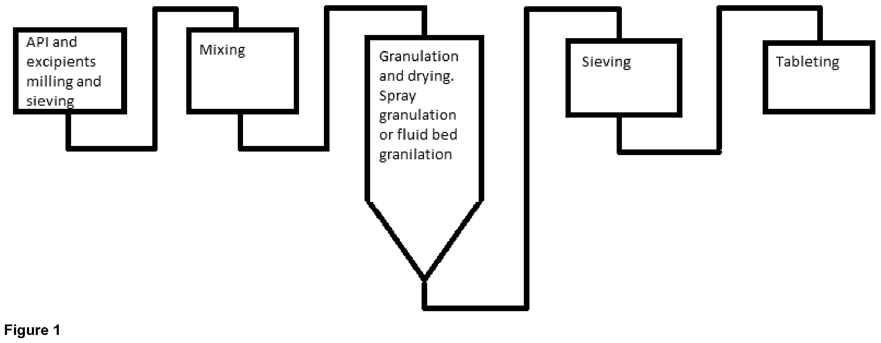

The content of water in a tablet is an essential feature for different properties of the tablet itself, such as compressibility, friability, hardness, disintegration time and others. However, the water content It is also important for the process of tableting, as it is for example very much depending on the water content whether e.g. a deficiency like capping is occurring during compression of the granules to a tablet.
Last but no least the water content is sometimes important for the stability of the active pharmaceutical ingredient in the tablet.
A general tableting process in the state of the art follows a scheme as depicted in figure 1. In a general tableting process, the active ingredients and excipients are milled and optionally sieved. Thereafter, the ingredients are mixed and a (lubrication-) liquid is added to the mixture for the granulation process. This may be performed by different techniques. For the wet granulation process, a specific grade of wetting is needed. The importance of the binder content in the wet granulation process is in detail described by Dave et al in Drug Dev. and Industrial Pharmacy, 2012, Vol. 38, Issue 4, pp 439-446.
The mixed granulate is then dried to the specific content of water before it is processed in a sieving process. In a last step addition of excipients and a final mixing step may be performed whenever excipients are employed that are not compatible with a high content of water.
State of the art apparatuses for the formation of compressible powders are closed systems. This is important for the safety of the operating and maintenance personnel and also for the high standards of the products quality. In these systems, the product transfer of the granules is achieved by vacuum transport systems.
In the state of the art, the content of water is adjusted in the drying process after granulation.
It is also reported that the value for loss on drying may be adjusted by storage in climated chambers with defined humidity. However, this method is requiring large climate chambers, takes time and needs GMP-controlled air. Although practical for small scale, the use of climate chambers for batches of more than 300 kg is limited.

### Summary of the invention

The inventors have now found that it is possible to rehumidify the granulated powder after sieving. Increase of the moisture content of the granulate after sieving improves the subsequent tableting process.

### Description of the invention

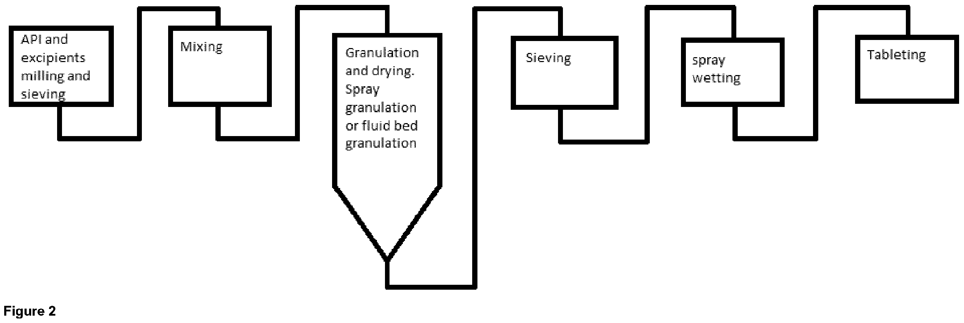

Figure 2 shows the new process for the manufacture of a tablet.
In a first step, the active pharmaceutical ingredient (API) is mixed with excipients, milled and sieved. In a further mixing step, the mixing is further processed, before the mixture is wetted with a wetting agent like water.
In a second step, granules are formed. For the formation of the granules, a specific moisture content is necessary. This moisture content may be different from the moisture content needed for the tablet pressing process.
After granulation, the mass is dried to a value that is ideal for the tableting process. The powder is then transported by vacuum - especially in a closed system - to the sieving process.
During transport and sieving a not predictable amount of water may evaporate from the mixture, thus forming a powder composition that is too dry for tableting.
In this case, it is necessary to rehydrate/ wet the composition in order to achieve the optimum water content for the next step.
As described above, wetting of a powder after the sieving step has been performed formerly by storage of the powder in a climate chamber with standardized humidity.
The inventors have now found that wetting of the powder may also be performed in a quick manner by spraying the particles while falling from an upper container into a lower container. Optionally, the wetted material may be blended in a container blender to homogenize the humidity.
In worst case the blend can optionally be additionally sieved (delumping) or is stored over night for equilibration).
The mixture is then used for the manufacture of tablets.

In one embodiment, the amount of water used for the wetting of granules that are used for the manufacture of tablets is calculated by the following formula:

$$W = G \times (T-V)$$

wherein

W = Needed Water for wetting [kg]
G = Weigh of granules after sieving [kg]
T = Target Value of water content before tableting [%]
V = Value of water content after sieving [%]

Examples:

Example 1 granulation and sieving of tableting mixture

| Material | Per Batch (kg) |
| --- | --- |
| Sitagliptin Sulfate | 6,203 |
| Metformin HCl | 100,000 |
| Microcryst. Cellulose | 13,124 |
| Copovidone K28 | 9,100 |
| EDTA Disodium Dihydrate-MT | 0,013 |
| Sodium Lauryl Sulfate | 0,260 |
| Sodium Stearyl Fumarate (Pruv) | 1,300 |

3.64 kg of Water is dispensed into a suitable mixing tank. Copovidone K28, Sodium Lauryl Sulfate and EDTA Disodium Dihydrate is added under stirring. Stirring is continued for about 2 hours. (Solution1)

Sitagliptin Sulfate and Metformin HCl are mixed in a shear mixer for 2 minutes at 80 rpm. (mixture 2) Moisture content of the mixture 2 at this stage is 0.26 %.

Solution 1 is now added to the dry mixture in a high shear mixer.

The wet granules are transferred to a fluid bed drier and dried to a moisture content of 2 %. The dried granulate is pumped to sieving using vacuum transfer and sieved through a 2 mm rasp sieve.

After sieving, the water content of the sieved mixture is at 1.2 %.

Example 2 wetting of dry granulate for tableting process

According to the actual content of water in the mixture and the water content needed for the tableting process, the difference of water is calculated.

Calculation: Target Value [2.0 %] – value from sieved granule = [%] amount of water for wetting.

Amount of water for wetting:
Weight of granules [kg] x calculated %-value = 113.4 kg x 0.8 % = 0.907 kg of Water.

The mixture is placed in an upper container that is connected via a lower valve to a lower container in a way that the mixture may fall from the upper container into the lower container if the valve is opened. Between the upper container and the lower container, a spray nozzle is placed in a way that powder falling from the upper container into the lower container must pass a curtain of water droplets, thus being wetted.

A pressure vessel containing the water for wetting is connected to the spray head unit. The granules are transferred from the top container to the bottom container while spraying the water from the pressure vessel on the granules during the transfer.

After wetting, excipients like microcrystalline cellulose is added to the mixture.

The mixture may optionally be blended and/ or sieved after the wetting process.

**Claims**

1. A process for manufacturing tablets containing at least one active pharmaceutical ingredient comprising the following steps:

   a) Milling and sieving of at least one ingredient
   b) Granulation of the mixture
   c) Optionally drying the mixture
   d) Transporting the mixture by vacuum transport
   e) Sieving the mixture
   f) Adjusting the moisture of the mixture by spraying the mixture with water
   g) Optionally sieving and / or blending of the mixture
   h) Use of the mixture for the manufacture of tablets

2. A process according to claim 1 wherein the adjustment of the moisture in step f) is performed while the mixture is falling from an upper container into a lower container.

3. A process according to claim 1 or 2 wherein the wetted mixture is further blended in a blending container.

4. A process according to one of the preceding claims wherein the used water is calculated according to the formula:

$$W = G \times (T-V)$$

wherein

   W = Needed Water for wetting [kg]
   G = Weigh of granules after sieving [kg]
   T = Target Value of water content before tableting [%]
   V= Value of water content after sieving [%]

**Patentansprüche**

1. Verfahren zur Herstellung von Tabletten, die mindestens einen pharmazeutischen Wirkstoff enthalten, umfassend die folgenden Schritte:

   a) Mahlen und Sieben von mindestens einem Bestandteil
   b) Granulierung des Gemischs
   c) Optional Trocknen des Gemischs
   d) Transportieren des Gemischs durch Vakuumtransport
   e) Sieben des Gemischs
   f) Einstellen der Feuchtigkeit des Gemischs durch Besprühen des Gemischs mit Wasser
   g) Optional Sieben und/oder Mischen des Gemischs
   h) Verwenden des Gemischs für die Herstellung von Tabletten.

2. Verfahren nach Anspruch 1, wobei die Einstellung der Feuchtigkeit in Schritt f) ausgeführt wird, während das Gemisch von einem oberen Behälter in einen unteren Behälter fällt.

3. Verfahren nach Anspruch 1 oder 2, wobei das angefeuchtete Mischung in einem Mischbehälter weiter gemischt wird.

4. Verfahren nach einem der vorstehenden Ansprüche, wobei das verwendete Wasser gemäß der folgenden Formel berechnet wird:

$$W = G \times (T\text{-}V)$$

wobei

W = Benötigtes Wasser zum Benetzen [kg]
G = Gewicht des Granulats nach Sieben [kg]
T = Zielwert des Wassergehalts vor Tablettierung [%]
V= Wert des Wassergehalts nach Sieben [%].

**Revendications**

1. Procédé de fabrication de comprimés contenant au moins un principe pharmaceutique actif comprenant les étapes suivantes :

   a) le broyage et le tamisage d'au moins un ingrédient
   b) la granulation du mélange
   c) le séchage éventuel du mélange
   d) le transport du mélange par transport sous vide
   e) le tamisage du mélange
   f) l'ajustement de l'humidité du mélange par pulvérisation du mélange avec de l'eau
   g) le tamisage et/ou le mélangeage éventuels du mélange
   h) l'utilisation du mélange pour la fabrication de comprimés.

2. Procédé selon la revendication 1, ledit ajustement de l'humidité à l'étape f) étant effectué pendant que le mélange est en train de tomber d'un récipient supérieur dans un récipient inférieur.

3. Procédé selon la revendication 1 ou 2, ledit mélange humide étant en outre mélangé dans un récipient de mélangeage.

4. Procédé selon l'une quelconque des revendications précédentes, ladite eau utilisée étant calculée selon la formule :

$$W = G \times (T\text{-}V)$$

dans laquelle

W = Eau nécessaire à l'humidification [kg]
G = Poids des granulés après tamisage [kg]
T = Valeur cible de la teneur en eau avant pastillage [%]
V = Valeur de la teneur en eau après tamisage [%].

Figure 1

| API and excipients milling and sieving | Mixing | Granulation and drying. Spray granulation or fluid bed granulation | Sieving | spray wetting | Tableting |

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **DAVE et al.** *Drug Dev. and Industrial Pharmacy,* 2012, vol. 38 (4), 439-446